(19) Europäisches Patentamt
European Patent Office
Office européen des brevets

(11) Numéro de publication: **0 168 282**
**B1**

(12) # FASCICULE DE BREVET EUROPEEN

(45) Date de publication du fascicule du brevet: **26.08.87**

(51) Int. Cl.⁴: **C 07 C 99/10**, C 07 C 102/08

(21) Numéro de dépôt: **85401086.5**

(22) Date de dépôt: **03.06.85**

**E R R A T U M**

| DIE TEXTSTELLE :<br>TEXT PUBLISHED :<br>LE PASSAGE SUIVANT : | (SEITE, SPALTE, ZEILE)<br>(PAGE, COLUMN, LINE)<br>(PAGE, COLONNE, LIGNE) | | | LAUTET BERICHTIGT:<br>SHOULD READ :<br>DEVRAIT ETRE LU : |
|---|---|---|---|---|
| sous le n° 2 518 973 | 2 | 1 | 48 | sous le n° 2 519 973 |

Tag der Entscheidung über die Berichtigung / Date of decision on rectification: / Date de décision portant sur modification: 13.11.87

Ausgabe- und Veröffentlichungstag: / Issue and publication date: / Date d'edition et de publication: 13.01.88

Patbl.Nr / EPB no: / Bull. no: 88/02

(19) Europäisches Patentamt
European Patent Office
Office européen des brevets

(11) Numéro de publication: **0 168 282**
**B1**

(12) # FASCICULE DE BREVET EUROPEEN

(45) Date de publication du fascicule du brevet: **26.08.87**

(51) Int. Cl.⁴: **C 07 C 99/10**, C 07 C 102/08

(21) Numéro de dépôt: **85401086.5**

(22) Date de dépôt: **03.06.85**

(54) **Procédé de synthèse en continu d'un alpha-amino-acide par hydrolyse catalytique chimique et dispositif pour la mise en oeuvre du procédé.**

(30) Priorité: **05.06.84 FR 8408762**

(43) Date de publication de la demande: **15.01.86 Bulletin 86/3**

(45) Mention de la délivrance du brevet: **26.08.87 Bulletin 87/35**

(84) Etats contractants désignés: **AT BE CH DE GB IT LI LU NL SE**

(56) Documents cités: **EP - A - 0 084 470**

(73) Titulaire: **CENTRE NATIONAL DE LA RECHERCHE SCIENTIFIQUE (CNRS), 15, Quai Anatole France, F-75007 Paris (FR)**

(72) Inventeur: **Commeyras, Auguste, Impasse des Ecoles Clapiers, F-34170 Castelnau Le Lez (FR)**
Inventeur: **Taillades, Jacques, 9, rue des Erables Lot. du Vert Pré Clapiers, F-34170 Castelnau Le Lez (FR)**
Inventeur: **Brugidou, Jean, 8, rue Jacques Boe La Paillade, F-34100 Montpellier (FR)**
Inventeur: **Mion, Louis, 477, rue d'Alco, F-34100 Montpellier (FR)**
Inventeur: **Sola, Régine Rés. Chêne Colombière, Bât. I 2, Res. Saint-Louis, Bat 2, 238, Ave d'Occitanie 34100 Montpellier (FR)**
Inventeur: **Pascal, Robert Rés. Chêne Colombière, Bât. I 2, Res. Saint-Louis, Bat 2, 238, Ave d'Occitanie 34100 Montpellier (FR)**
Inventeur: **Lasperas, Monique, 793 Bd de la Lironde Montferrier sur Lez, F-34980 St. Gely Du Fesc (FR)**
Inventeur: **Rousset, Alain, Orée du Bois 171, avenue d'Occitanie, F-34100 Montpellier (FR)**

(74) Mandataire: **Ahner, Francis et al, CABINET REGIMBEAU 26, avenue Kléber, F-75116 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention se rapporte à un procédé de synthèse d'un α-amino-acide, ou de l'un de ses sels, par hydrolyse catalytique chimique.

De façon particulière, la présente invention a pour objet un procédé de synthèse en continu d'α-amino-acides par hydrolyse catalytique chimique à partir d'α-amino-nitriles, ou leurs sels, correspondants.

La présente invention concerne également un dispositif permettant la mise en œuvre de ce procédé de synthèse.

Il convient à ce propos de rappeler que les α-amino-acides, en particulier les α-amino-acides dits essentiels, revêtent un intérêt industriel incontestable et sans cesse croissant. Certains d'entre eux peuvent en effet être utilisés en médecine humaine ou animale, notamment pour pallier certaines carences alimentaires en protéines animales dans les pays en voie de développement, ou pour la supplémentation des protéines végétales utilisées pour la nourriture du bétail ou des volailles. D'autres de ces composés peuvent également entrer par exemple dans la composition de savons ou de cosmétiques.

Dans la technique antérieure, les α-amino-acides sont préparés à partir de leurs précurseurs aldéhydiques correspondants, par la réaction de Strecker ou encore par une des nombreuses modifications apportées à la réaction originale, et en particulier par la mise en œuvre du procédé décrit dans le brevet français n° 2372797. Conformément au procédé de ce brevet antérieur, l'α-amino-nitrile intermédiaire ou l'un de ses sels est hydrolysé catalytiquement en milieu basique, en faisant réagir une solution aqueuse contenant au moins un dérivé carbonylé sur ledit α-amino-nitrile ou sur l'un de ses sels, en présence d'ions hydroxydes. Un tel procédé, extrêmement sélectif et économiquement favorable, nécessite cependant la séparation du catalyseur carbonylé du milieu réactionnel et son éventuel recyclage. De plus, dans ce procédé antérieur, le choix du catalyseur carbonylé se trouve fortement limité par sa nécessaire stabilité en milieu basique homogène.

La demande de brevet français publiée sous le n° 2518973 décrit un procédé de préparation d'α-amino-acides permettant d'éviter en partie les inconvénients qui viennent d'être signalés. En effet, selon ce procédé antérieur, l'hydrolyse catalytique chimique de l'α-amino-nitrile de départ ne s'effectue plus en phase homogène, mais en phase hétérogène. Pour ce faire, on utilise un catalyseur carbonylé non plus en solution dans le milieu réactionnel, mais une résine polymère appropriée, comportant des groupements carbonyles. Conformément à ce procédé antérieur, on constate qu'en introduisant un α-amino-nitrile ou l'un de ses sels, dans une solution aqueuse contenant une suspension d'une résine polymère carbonylée insoluble et des ions hydroxydes, on obtient très rapidement, l'α-amino-amide correspondant à l'α-amino-nitrile de départ. La résine carbonylée insoluble en milieu basique aqueux peut alors être séparée du milieu réactionnel par simple filtration ou centrifugation, puis être recyclée sans qu'un processus de régénération soit nécessaire. De plus, au lieu d'être effectué de façon discontinue, avec recyclage du catalyseur, l'emploi d'une résine polymère carbonylée permet la mise en œuvre du procédé de façon continue par contact d'une solution d'α-amino-nitrile ou de l'un de ses sels avec le catalyseur carbonylé immobilisé.

Cependant, la mise en œuvre d'un tel procédé de préparation d'α-amino-acides par hydrolyse catalytique chimique *en continu* se révèle beaucoup plus délicate qu'il n'y paraît au premier abord. En particulier, on observe une diminution progressive de l'activité catalytique de la résine due à un empoisonnement des groupements carbonylés terminaux. Cette décroissance sensible de l'activité catalytique limite considérablement l'application industrielle d'un tel procédé et constitue un obstacle majeur à la généralisation de celui-ci dans la synthèse des α-amino-acides dont le marché internationl est en pleine expansion.

La présente invention a précisément pour but de remédier à cet inconvénient relatif à l'empoisonnement du catalyseur carbonylé.

C'est pourquoi la présente invention concerne un procédé de synthèse en continu d'un α-amino-acide par hydrolyse catalytique chimique en milieu basique aqueux, d'un α-amino-nitrile ou d'un de ses sels, en présence d'au moins un dérivé carbonylé, comprenant:

a) une première étape d'hydratation catalytique de l'α-amino-nitrile de départ ou l'un de ses sels, en l'α-amino-amide correspondant, en présence d'une faible concentration d'ions hydroxydes et en utilisant en tant que catalyseur une résine polymère carbonylée insoluble en milieu basique aqueux,
b) une deuxième étape d'hydrolyse de l'α-amino-amide ainsi formé en α-amino-acide sous la forme d'un de ses sels correspondant en présence d'ions hydroxydes en concentration sensiblement équimolaire par rapport à la concentration de l'α-amino-amide,

caractérisé en ce qu'on prélève une fraction du volume du milieu réactionnel de la deuxième étape en vue de son recyclage, après refroidissement, vers le milieu réactionnel de la première étape, ladite fraction étant déterminée de façon à assurer la dilution de l'α-amino-nitrile ou l'un de ses sels, introduit dans le milieu de la première étape, et à maintenir en dessous du seuil d'empoisonnement de la résine polymère carbonylée la concentration en α-amino-amide du milieu réactionnel de la première étape, ledit seuil d'empoisonnement étant égal à environ 0,20 M/l d'α-amino-amide.

Il apparaît en effet que deux paramètres — concentration en ions hydroxydes au niveau de chacune des deux étapes du procédé et concentration en α-amino-amide dans le milieu réactionnel de la première étape — interviennent de façon déterminante dans le procédé selon l'invention.

Des études approfondies ont permis à la demanderesse de mieux comprendre le mécanisme d'empoisonnement de la résine carbonylée par l'α-

amino-amide formé et d'établir les valeurs de la concentration en α-amino-amide compatible avec une synthèse en continu de l'α-amino-acide que l'on désire préparer.

Selon la présente invention, un procédé de synthèse d'un α-amino-acide, sous forme de l'un de ses sels, à une concentration de xM à partir d'un α-amino-nitrile, ou d'un de ses sels, à la même concentration xM, est caractérisé en ce que la fraction du volume du milieu réactionnel de la deuxième étape, prélevée et recyclée, est déterminée de manière que la concentration en α-amino-amide, dans le milieu de la première étape, soit sensiblement de $\frac{x}{10}$ M.

De façon plus particulière, un procédé de synthèse d'un α-amino-acide, sous forme de l'un de ses sels, à une concentration de 1 M à partir d'un α-amino-nitrile, ou d'un de ses sels, à la même concentration 1 M, selon la présente invention, est caractérisé en ce que la fraction du volume du milieu réactionnel de la deuxième étape, prélevée et recyclée est déterminée de manière que la concentration en α-amino-amide, dans le milieu réactionnel de la première étape, soit sensiblement décimolaire.

Selon une autre caractéristique du procédé de la présente invention, les ions hydroxydes sont introduits dans le milieu réactionnel de la deuxième étape à raison d'une quantité sensiblement équimolaire par rapport à l'α-amino-amide issu de la première étape.

Selon une autre caractéristique du procédé de la présente invention, la quantité d'ions hydroxydes nécessaires à la catalyse dans la première étape provient du recyclage d'une fraction du volume réactionnel de la deuxième étape.

Les études ayant permis d'établir les conditions optimales qui viennent d'être définies, pour la mise en œuvre du procédé de synthèse en continu selon la présente invention sont rapportées ci-après.

En ce qui concerne les résines polymères carbonylées appropriées à la mise en œuvre de ce procédé, on se référera, sans pour autant que celles-ci soient limitatives, aux résines polymères porteuses de chaînes latérales à groupement carbonylé terminal, ainsi que leurs procédés de préparation respectifs, décrites dans la demande de brevet français n° 2 519 973.

L'activité catalytique de ces résines a été testée dans un montage en continu. Suite à ces essais préliminaires, on a retenu la résine poly-N-acyl pipéridone dont le motif actif est illustré par la formule I:

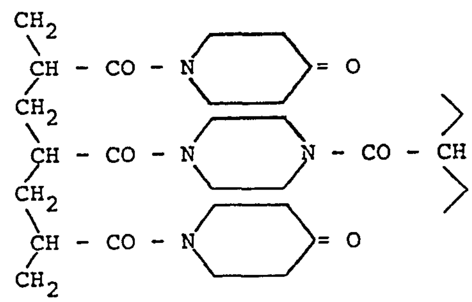

(I)

Cette résine est obtenue par polymérisation d'acryloylpipéridone:

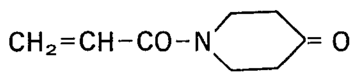

(80%) et, réticulée avec 20% de N,N'-bis-acryloylpipérazine:

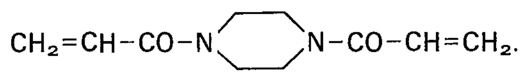

Elle s'est avérée très efficace (capacité # 2,5 méq./q, après conditionnement en milieu basique), en outre sa préparation est relativement facile.

Le mécanisme de la catalyse est représenté de façon simplifiée ci-après sur le schéma 1. La réaction se réduit cinétiquement à deux étapes, d'une part la formation de l'intermédiaire imine et d'autre part, l'hydrolyse de l'imine avec régénération du catalyseur qui est l'étape cinétiquement déterminante.

Pour des commodités d'analyse, notamment en RMN, on a étudié le mécanisme de cette catalyse en utilisant essentiellement comme substrat l'α-aminopropionitrile – formule II. Ce composé est cepenant représentatif de la réactivité de l'α-aminométhylmercaptobutyronitrile – formule III – précurseur de la méthionine.

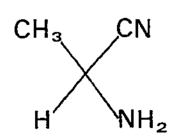

(II)

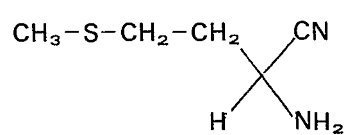

(III)

*SCHÉMA 1:* Mécanisme du processus catalytique d'hydratation.

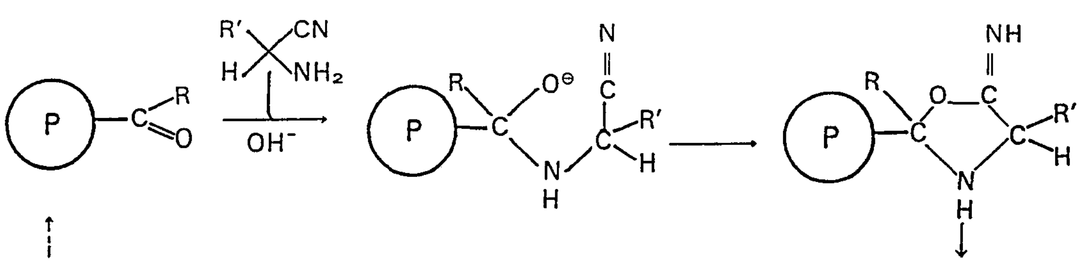

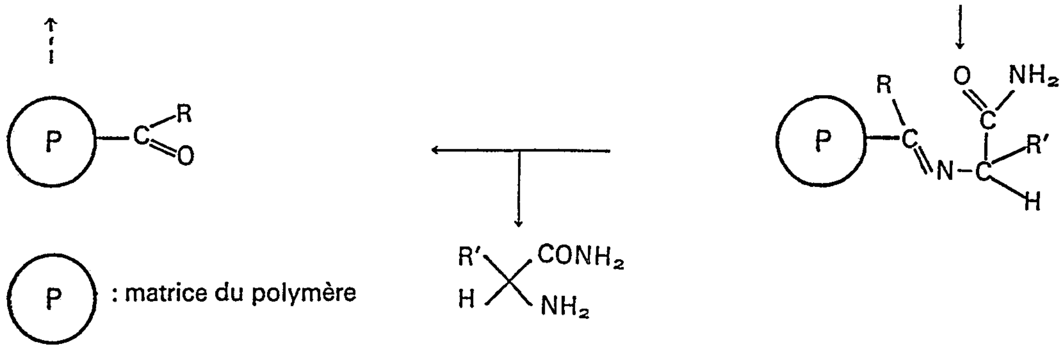

: matrice du polymère

L'interprétation du processus catalytique sur résine carbonylée, qui est assorti d'un comportement cinétique complexe, repose sur le principe d'une distribution de réactivité, qui traduit la non-équivalence des sites catalytiques fixés sur le polymère.

On observe qu'à la concentration 0,1 molaire en α-aminonitrile, le catalyseur (I) présente une activité catalytique satisfaisante, notamment supérieure à celle de l'acétone. Dans ces conditions, et en utilisant une concentration en soude de l'ordre de 0,1 mole/l et à 25° C, le catalyseur transforme quantitativement l'α-aminonitrile en α-aminoamide en ne subissant aucun vieillissement notable.

Pour des concentrations molaires en α-aminonitrile, on observe une diminution progressive de l'activité catalytique. L'origine de cet empoisonnement du catalyseur correspond à la formation de l'imidazolidinone-4, selon un mécanisme qui dépend de la nature du composé carbonylé. Dans le cas particulier des composés cétoniques, l'imidazolidinone se forme à partir de l'intermédiaire imine suivant le schéma réactionnel:

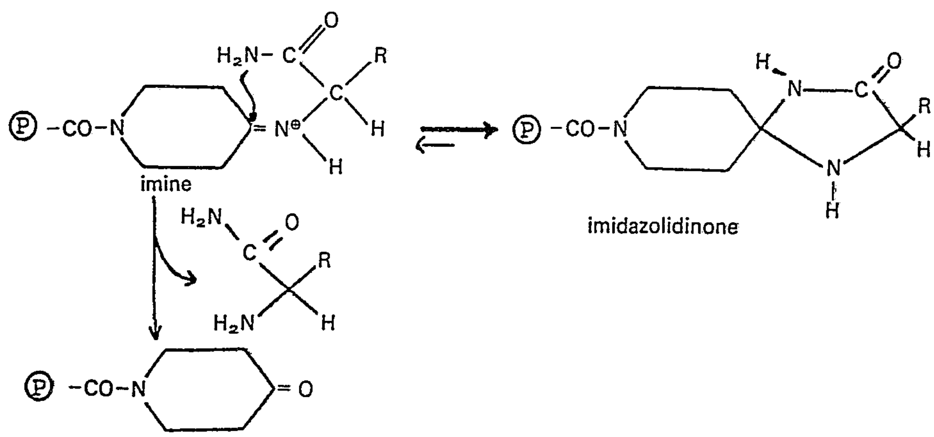
imine
imidazolidinone

où (P) représente la matrice du polymère.

Cette réaction secondaire est favoriée par l'accroissement de la concentration en α-aminonitrile qui conduit à l'accumulation de l'imine de l'α-aminoamide sur les sites catalytiques de la résine.

La réaction étant équilibrée l'empoisonnement du catalyseur est réversible. Ainsi, on peut par exemple régénérer une résine ainsi inactivée en faisant circuler sur cette dernière de l'eau à 80° C. L'imidazolidinone est dans ces conditions hydrolysée en α-aminoamide tandis que la résine recouvre son activité catalytique initiale.

Pour dégager les conditions optimales d'utilisation du catalyseur il convient donc de tenir compte des impératifs liés non seulement au processus catalytique lui-même, mais également à ceux afférents aux réactions secondaires à savoir, la réaction autocatalytique et la formation de l'imidazolidinone.

Lors de la réaction d'hyratation, l'étape lente est l'hydrolyse de l'imine; son énergie d'activation est de l'ordre de 16 kcal/mole, c'est-à-dire nettement supérieure à celle de la première étape, où elle n'est globalement que de 3 kcal/mole.

Par conséquent, pour éviter la saturation des sites catalytiques et accroître le «turn over» du catalyseur, il semble logique d'augmenter la température réactionnelle pour accélérer la 2e étape. En outre, ceci serait en faveur d'une augmentation du taux de forme carbonylée par rapport à la forme hydratée et corollairement d'un accroissement de réactivité.

On ne peut toutefois s'en tenir à cette seule observation en effet d'autres phénomènes interviennent.

En premier lieu, dans les conditions basiques requises pour l'hydratation de l'α-aminonitrile ce dernier se décompose lentement ($k_D = 3{,}3\ 10^{-3}\ mn^{-1}$ pour $R' = CH_3$) selon la réaction:

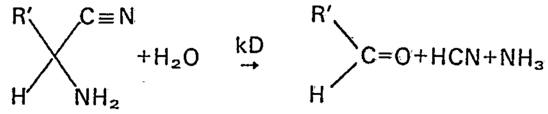

indépendante du pH, mais fortement accélérée par une élévation de température ($E_A$=23 kcal/Mol pour $R'=CH_3$).

L'aldéhyde apparaissant dans le milieu, peut, en raison de sa grande réactivité, jouer le rôle de catalyseur d'hydratation du nitrile

($k'_H=4.10^{-3}$ $mn^{-1}$ $moles^{-2}$ $l^2$ pour $R'=CH_3$).

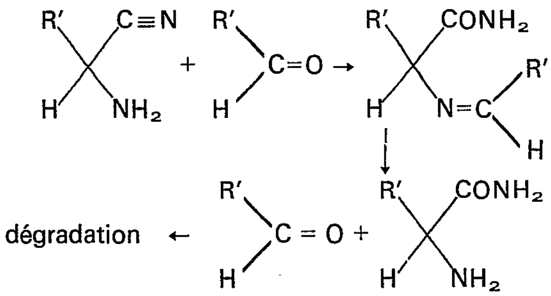


Contrairement aux apparences, cette réaction est néfaste car l'aldéhyde, en milieu basique, se dégrade rapidement par diverses réactions (addition sur les amines présentes dans le milieu, aldolisation-crotonisation) en abaissant considérablement le rendement global de la réaction. (70% dans le cas de l'α-aminométhyl mercaptobutyramide). Il est donc nécessaire de supplanter la réaction autocatalytique par le processus catalytique, donc d'opérer dans des conditions d'efficacité maximale de la colonne, c'est-à-dire de telle sorte que le rapport ε soit le plus grand possible.

$$\epsilon = \frac{\text{nombre de sites catalytiques}}{\text{nombre de molécules de substrat}}$$

$= \frac{m.CP_{c=o}}{Vr.}$ 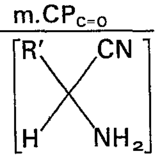

m: masse de résine

$Cp_{C=O}$: capacité de la résine

Vr: volume résiduel de la colonne (occupé par la solution)

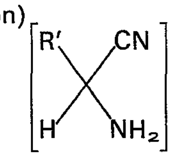 = concentration de la solution injectée en aminotrile.

Pour une colonne de résine donnée, correctement remplie, la seule possibilité d'augmenter le rapport ε est de diminuer la concentration en α-aminonitrile.

Il découle de cette observation que l'on ne peut opérer avec une concentration en α-aminonitrile élevée. Cette limitation rejoint celle imposée par la réaction de formation de l'imidazolidinone, qui implique de ne pas utiliser une forte concentration en α-aminoamide, donc en α-aminonitrile qui est son précurseur.

Il ressort donc de cette analyse des conditions d'utilisation des catalyseurs carbonylés supportés:

Premièrement, que la température réactionnelle doit être limitée. Un compromis acceptable se situe aux alentours de 30° C.

Deuxièmement que la concentration en α-aminonitrile ne doit pas excéder une valeur de l'ordre de 0,2 mole/litre, sous peine de favoriser les réactions secondaires, avec comme conséquence une diminution du rendement de la réaction et un empoisonnement du catalyseur, qui bien que réversible est tout de même à éviter.

Ces conditions d'utilisation du catalyseur sont en opposition avec un impératif d'ordre économique qui impose d'opérer sur des solutions d'α-aminonitriles relativement concentrées, afin d'obtenir au stade final l'α-aminoacide à une concentration acceptable de l'ordre de 1 mole/litre. Il importe en effet d'éviter de coûteuses opérations de concentration et de limiter les dimensions de l'installation.

C'est pour pallier ces difficultés et concilier les impératifs d'ordres réactionnels et économiques que la présente invention propose une solution technologique consistant à diluer sur la colonne de catalyse l'α-aminonitrile avec une solution de l'α-amino-acide correspondant. Le principe de ce procédé de synthèse en continu des α-amino-acides est représenté sur la figure annexée qui illustre schématiquement un mode de réalisation particulier de l'installation selon l'invention. Les débits, concentrations et températures qui apparaissent sur cette figure correspondent à la préparation de l'alanine.

Ces études relatives à ce principe de synthèse en continu d'α-amino-acides par hydrolyse catalytique chimique ont abouti à la mise au point d'un dispositif permettant de mettre en œuvre, de façon optimale, ce procédé.

C'est pourquoi un autre objet de la présente invention se rapporte à un dispositif pour la mise en œuvre du procédé de synthèse en continu d'un α-amino-acide, ledit dispositif comportant:

a) une cuve d'alimentation 10 en α-amino-nitrile, ou en l'un de ses sels,
b) un conduit de raccordement 12 sur lequel est montée une pompe 14, reliant la cuve d'alimentation 10 à
c) une colonne de catalyse 16 contenant une résine polymère carbonylée 18 insoluble en milieu basique aqueux,
d) un conduit de raccordement 20 de la colonne de catalyse 16 à
e) un réacteur d'hydrolyse 22, faisant simultanément office de réservoir pour le sel d'α-amino-acide formé, comportant des moyens de chauffage et d'agitation du milieu réactionnel, et muni
f) de moyens d'alimentation en ions hydroxydes comprenant une cuve d'alimentation 24, un conduit 26 et une pompe 28,
g) d'un conduit de soutirage 30 du sel d'α-amino-acide formé,

caractérisé en ce que ledit dispositif comporte, en outre, un conduit de recyclage 32 équipé d'un réfrigérant 34 et d'une pompe 36, ledit conduit de recyclage raccordant le réacteur d'hydrolyse 22 à la colonne de catalyse 16 afin d'assurer la dilution du courant d'α-amino-nitrile, ou d'un de ses sels, alimentant ladite colonne de catalyse.

Selon une autre caractéristique dudit dispositif, les pompes 14, 28 et 36, respectivement montées sur les conduits

- d'alimentation en α-amino-nitrile, ou l'un de ses sels 12,
- d'alimentation en ions hydroxydes 26, et
- de recyclage 32 du milieu réactionnel de la deuxième étape,

sont des pompes centrifuges à débits réglables.

Selon une autre caractéristique de ce même dispositif, le réacteur d'hydrolyse 22 possède en outre un conduit 38 débouchant dans le ciel gazeux dudit réacteur et destiné à recycler l'ammoniac dégagé lors de la deuxième étape en vue de son utilisation pour la préparation in situ de l'α-amino-nitrile de départ, ou l'un de ses sels.

Selon une autre caractéristique, le dispositif de la présente invention, comporte au moins une deuxième colonne de catalyse montée en parallèle par rapport à la première colonne 16 pour fonctionner en alternance par rapport à celle-ci, chacune de ces colonnes étant équipée de moyens aptes à régénérer la résine polymère carbonylée, et d'un système de vannes permettant le fonctionnement alternatif des colonnes de catalyse.

Les moyens aptes à régénérer la résine polymère carbonylée comportent un circuit de lavage à l'eau comprenant (i) une arrivée d'eau chauffée à environ 80° C, (ii) une colonne de régénération dans laquelle est disposée une résine, sulfonique pour fixer l'amide entraînée dans ledit circuit, ladite colonne de régénération étant raccordée par (iii) des conduits de raccordement à l'entrée et à la sortie de la colonne de catalyse.

La résine sulfonique peut elle-même être régénérée par une élution à l'ammoniaque.

Le fonctionnement détaillé sera développé ci-après, mais d'une manière simplifiée, il se déroule de la façon suivante:

L'α-aminonitrile est stocké à la concentration de 1 mole/litre en présence d'ammoniaque 5M, et à basse température (0° C), ce qui assure sa stabilisation thermodynamique optimale.

Il est introduit en continu dans la colonne de catalyse d'hydratation remplie de résine carbonyle, après avoir été dilué par recyclage de l'α-amino-acide en solution basique. En sortie de colonne l'α-amino-amide formé par hydratation du nitrile et dont la concentration $Am_2$ est de l'ordre de 0,1 mole/l est introduit dans le réacteur d'hydrolyse porté à 80° C où il est hydrolysé en α-amino-acide. La soude nécessaire à l'hydrolyse est ajoutée en continu en solution très concentrée et à très faible débit. L'ammoniac, d'une part introduit dans le réacteur, et d'autre part formé par hydrolyse de l'amide, peut être facilement récupéré et réutilisé pour synthétiser, avec l'aldéhyde et l'acide cyanhydrique, l'α-amino-nitrile.

L'α-amino-acide produit, sous forme de sel, est soutiré du réacteur d'hydrolyse à un débit pratiquement égal à celui de l'α-amino-nitrile introduit (si l'on néglige le débit extrêmement faible de la solution de soude additionnée). La concentration de l'α-amino-acide ainsi synthétisé est de l'ordre de 1 mole/litre.

A titre d'illustration, on mentionnera ci-après quelques exemples particuliers de synthèse en continu d'α-amino-acides par le procédé d'hydrolyse catalytique chimique, et en utilisant l'installation selon la présente invention.

*EXEMPLE 1:*

*PRÉPARATION D'ALANINE EN CONTINU À PARTIR DE L'α-AMINO-PROPIONITRILE*

A — *DÉTERMINATION DES CONDITIONS OPÉRATOIRES:*

Les résultats de l'étude fondamentale approfondie du processus de transformation d'un aldéhyde en l'acide α-aminé correspondant, tant au niveau des mécanismes réactionnels, que des différents paramètres cinétiques, ont permis de prévoir les valeurs des différents paramètres (débits, concentrations, températures) conditionnant le bon fonctionnement de l'installation pilote.

1/ *Réaction d'hydratation* (catalyse)

On utilise une colonne (hauteur: 250 mm, diamètre: 6 mm) thermostatée à 30° C, contenant 1,5 g de résine N-acyl pipéridone (I), ce qui correspond à 5,5 g de résine mouillée. Le volume résiduel Vr est de 5 $cm^3$ environ. La capacité de la résine étant de 2,5 meq./g, la colonne comporte donc environ 3,7 milliéquivalents de groupements cétoniques accessibles et efficaces.

La réaction d'hydratation de l'α-amino-nitrile est sous contrôle chimique; la vitesse de dispersion de l'α-aminonitrile est proportionnelle au nombre de sites carbonylés du catalyseur, et du 1er ordre par rapport aux concentrations en α-amino-nitrile et en ions hydroxyles.

Pour l'ensemble de conditions suivantes:

- 1,5 g de résine pipéridone (I)
- $OH^- = 0,15M$
- 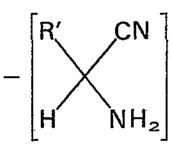 = 0,10M
- volume de la solution: 5 $cm^3$
- t = 20° C.

Le temps de ½ vie de la réaction de disparition de l'α-amino-nitrile déterminé en «batch» est de l'ordre de la minute. On peut donc considérer que la disparition du nitrile sera totale en 3 minutes soit 3 × t½.

On peut transposer ces résultats au fonctionnement continu de la colonne. La concentration en α-aminonitrile en sortie de colonne «C» est reliée à la concentration en α-aminonitrile en tête de colonne «Co» par la relation:

$$C = Co.e^{-K\tau}$$

τ: temps séjour dans la colonne

K = cte de vitesse expérimentale.

On peut déterminer approximativement le débit «d» d'écoulement du nitrile dans la colonne pour avoir une hydratation totale.

$$\tau = \frac{V}{d} \qquad d = \frac{5}{1,5} = 3,3\ cm^3/min.$$

L'utilisation d'un débit de 1 $cm^3$/min permet d'avoir une marge de sécurité suffisante pour tenir compte en particulier de l'occupation partielle des sites catalytiques par l'imine de l'α-aminoamide.

2/ *Réaction d'hydrolyse*

La vitesse d'hydrolyse de l'α-amino-amide en α-amino-acide en milieu basique est du premier ordre par rapport aux concentrations en amide et en ions hydroxyles. L'énergie d'activation de la réaction est de 13,5 Kcal/mole et la constante de vitesse $K = 2{,}9.10^{-2}\ M^{-1}$ à 35° C. Le volume V du réacteur d'hydrolyse peut être déterminé à partir de la balance massique d'entrée et de sortie de l'α-amino-amide dans le réacteur fonctionnant en régime stationnaire. Les variations intervenant dans les équations ci-dessous sont définies dans le schéma:

$$\underset{\text{amide introduit}}{d_2[Am]_2} = \underset{\text{amide résiduel recyclé}}{d_3[Am]} + \underset{\text{amide hydrolysé}}{V.k\,[OH^-]\,[Am]}$$

$$\text{soit: } d_2\frac{[Am]_2}{[Am]} = d_3 + V.k[OH^-]$$

$$V = d_2\frac{[Am]_2}{[Am]} - d_3 \cdot \frac{1}{K\,[OH^-]}$$

Si l'on fixe à priori un taux de conversion de 90%

$$\frac{[Am]_2}{[Am]} = 10$$

Les différents paramètres intervenant dans l'équation peuvent être déterminés comme suit:

a) *débits:*

Suite au calcul de temps de séjour dans la colonne de catalyse, le débit a été fixé à 1 ml/minute.

Donc: $d_2 = 1$ ml/min.

Par ailleurs, le rapport

$$\frac{d_o}{d_2}$$

est choisi égal à 1/10 de façon que la concentration en nitrile dans la colonne de catalyse soit 0,1 molaire par conséquent $d_o = 0{,}1$ ml/min.

Le débit de recyclage sera donc $d_3 = d_2 - d_o$ $d_3 = 0{,}90$ ml/min.

b) *Détermination de la concentration de la solution de soude dans le réacteur d'hydrolyse:*

Le bilan massique de la soude à l'entrée et à la sortie de la colonne de catalyse peut s'écrire sous la forme:

$$[d_2 - d_o]\ [OH^-] = d_2\,[OH^-]_2$$

$$\text{d'où: } \boxed{[OH^-] = \frac{d_2}{d_2 - d_o}[OH^-]_2}$$

La valeur est fixée au départ: $[OH^-]_2 = 0{,}15$M/l par suite:

$$[OH^-] = 0{,}15 \times \frac{1}{1 - 0{,}1} = 0{,}166 \text{ mole/l}$$

c) *Volume du réacteur d'hydrolyse:*

Afin de limiter le volume du réacteur d'hydrolyse, on a choisi d'opérer à 80° C (cette valeur n'est pas limitative). Dans ces conditions, la constante de vitesse d'hydrolyse K est de l'ordre de $0{,}36\ M^{-1}\ min^{-1}$.

Le volume du réacteur est donné par l'expression précédemment établie:

$$\boxed{V = \left[d_2 \cdot \frac{[Am]_2}{[Am]} - d_3\right] \cdot \frac{1}{k\,[OH^-]}}$$

$$V = [1 \times 10 - 0{,}9] \times \frac{1}{0{,}36 \times 0{,}166} = 152\ cm^3$$

$$V = 152\ cm^3$$

d) *Concentration en amide résiduel en sortie du réacteur d'hydrolyse:*

Les relations suivantes expriment la balance massique concernant l'α-amino-amide respectivement dans la colonne de catalyse et dans le réacteur d'hydrolyse.

$$\underset{\text{nitrile introduit}}{d_o x_o} + \underset{\text{amide recyclé}}{(d_2 - d_o)\,[Am]} = \underset{\text{amide formé}}{d_2\,[Am]_2}$$

$$\underset{\text{amide introduit}}{d_2\,[Am]_2} = \underset{\text{amide recyclé}}{d_3\,[Am]} + \underset{\text{acide formé}}{Vk\,[OH^-]\,[Am]}$$

en les égalant, on obtient:

$$d_3\,[Am] + Vk\,[OH^-]\,[Am] = d_o x_o = (d_2 - d_o)\,[Am]$$

$$[Am] = \frac{d_o x_o}{d_3 - d_2 + d_o + Vk\,[OH^-]}$$

mais $d_3 = d_2 + d_1$

$$\boxed{[Am] = \frac{d_o x_o}{(d_o + d_1) + Vk\,[OH^-]}}$$

on fixe pour $d_1$ une valeur très faible 0,01/min. pour éviter la dilution du milieu.

$$[Am] = \frac{0{,}1 \times 1}{(0{,}1 + 0{,}01) + 152 \times 0{,}36 \times 0166}$$

$$[Am] = 0{,}01 \text{ mole/l}$$

e) *Calcul de la concentration de la solution de soude ajoutée en continu:*

A partir des relations suivantes exprimant le bilan massique de la soude à l'entrée et à la sortie respectivement de la colonne de catalyse et du réacteur d'hydrolyse.

$$\underset{\text{soude introduite}}{(d_2 - d_o)\,[OH^-]} = \underset{\text{soude sortante}}{d_2\,[OH^-]_2}$$

$$\underset{\text{soude ajoutée}}{d_1\,[OH]_1} + \underset{\text{soude entrante}}{d_2\,[OH^-]_2} = \underset{\text{soude recyclée}}{d_3\,[OH^-]}$$

$$+ \underset{\text{soude consommée}}{V.k.\,[OH^-]\,[Am]}$$

$$d_1\,[OH^-]_1 + (d_2 - d_o)\,[OH^-] = d_3\,[OH^-] + V.k\,[OH^-]\,[Am]$$

$$d_3 = d_1 + d_2$$

$$d_1\,[OH^-]_1 = (d_1 - d_2) - (d_2 - d_o)\,[OH^-] + V\,k\,[OH^-]\,[Am]$$

$$d_1\,[OH^-]_1 = [OH^-]\,(d_1 + d_o) + V.k.\,[Am]$$

$$\boxed{[OH^-]_1 = \frac{[OH^-]}{d_1}(d_1 + d_o) + Vk\,[Am]}$$

$$[OH^-]_1 = \frac{0{,}166}{0{,}01}\,[(0{,}01 + 0{,}1) + 152 \times 0{,}36 \times 0{,}01]$$

$$[OH^-]_1 = 10{,}9 \text{ moles/litres}$$

f) *Evaluation de la concentration en ammoniac dans le réacteur d'hydrolyse:*

Comme précédemment, on écrit le bilan massique de l'ammoniac respectivement dans la colonne de catalyse et dans le réacteur d'hydrolyse.

$$d_o\,[NH_3]_o + (d_2 - d_o)\,[NH_3] = d_2\,[NH_3]_2$$

$$V.k.\,[OH^-]\,[Am] + d_2\,[NH_3]_2 = d_3\,[NH_3]$$

en combinant ces deux relations, il vient:

$$(d_3 - d_2 + d_o)\,[NH_3] - d_o\,[NH_3]_o = V.k.\,[OH^-]\,[Am]$$

$$(d_1 + d_o)\,[NH_3] - d_o\,[NH_3]_o = V.k.\,[OH^-]\,[Am]$$

$$\boxed{[NH_3] = \frac{1}{d_1 + d_o}\,(d_o\,[NH_3]_o + V.k.\,[OH^-]\,[Am])}$$

$$[NH_3] = \frac{1}{0{,}1 + 0{,}01}\,(0{,}1 \times 5 + 152 \times 0{,}36 + 0{,}166 \times 0{,}01)$$

$$[NH_3] = 5{,}36 \text{ moles/litre.}$$

h) *Evaluation de la concentration en acide α-aminé formé:*

Le bilan massique de l'α-amino-acide dans le réacteur d'hydrolyse et dans la colonne de catalyse s'exprime respectivement par les relations suivantes:

$$(d_2 - d_o)\,[Ac] = d_2\,[Ac]_2$$

$$V.k.\,[OH^-]\,[Am] + d_2\,[Ac]_2 = d_3\,[Ac]$$

moyennant ces deux relations on peut écrire:

$$(d_3 - d_2 + d_o)\,[Ac] = V.k.\,[OH^-]\,[Am]$$

par ailleurs, il a précédemment été établi que:

$$V.k.\,[OH^-]\,[Am] = d_o x_o - (d_o - d_1)\,[Am]$$

en outre $d_3 = d_2 + d_1$
on a donc:

$$(d_1 + d_o)\,[Ac] = d_o x_o - (d_o + d_1)\,[Am]$$

$$\boxed{[Ac] = \frac{d_o x_o}{d_1 + d_o} - [Am]}$$

$$[Ac] = \frac{0{,}1 \times 1}{0{,}01 + 0{,}1} - 0{,}01 = 0{,}899 \simeq 0{,}90 \text{ mole/l.}$$

Dans les conditions opératoires retenues la concentration de l'acide amine formé est égale à 90% de la concentration de l'α-amino-nitrile introduit.

L'α-amino-acide est soutiré à un débit D: $d_o + d_1 = 0{,}11$ ml/minute.

i) *Contrôle de fonctionnement de l'installation:*

Le contrôle du fonctionnement est réalisé à différents niveaux, et ce par plusieurs méthodes.

Les effluents de la colonne de catalyse sont analysés par RMN. On vérifie dans la série de l'alanine que l'hydratation de l'α-amino-nitrile en amide est totale. Au pH auquel on opère, les signaux du groupement méthyle de l'acide aminé, de l'α-amino-nitrile et de l'α-amino-amide sont en effet séparés.

De même, on contrôle par RMN le fonctionnement du réacteur d'hydrolyse.

La pureté de l'α-amino-acide est également contrôlée par CCM ($SiO_2$ éluant; propanol-$_2$/ammoniaque 34%: 70/30 – révélateur: ninhydrine).

Au moyen d'un titrateur potentiométrique, on dose par HCl (1 N), sur des prises d'essai de 1 $cm^3$ diluées avec 50 $cm^3$ de méthanol, successivement: les ions hydroxyles $[OH^-]$, l'ammoniac $[NH_3]$, la fonction amine de l'acide aminé, puis les groupements carboxyliques de l'α-amino-acide. On peut également chasser au préalable l'ammoniac. Le fait d'obtenir:

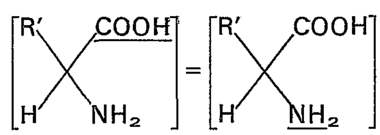

constitue un critère de pureté supplémentaire.

B – *SYNTHÈSE EN CONTINU D'ALANINE:*

Le réacteur d'hydrolyse est chargé avec 150 $cm^3$ d'une solution:

0,88M en alaninate de sodium,
0,166M en soude,
5,4M en ammoniaque,

de façon à atteindre immédiatement l'état stationnaire. Il est porté à 80-85° C.

L'α-amino-propionitrile 1 M, en solution ammoniacale 5M, stocké à 0° C, est injecté avec un débit $x_o = 0{,}10$ ml/m. Il est dilué à l'entrée de la colonne de catalyse par la solution d'α-amino-acide basique recyclé à un débit de 0,9 ml/min. La soude 10,9M alimente le réacteur d'hydrolyse avec un débit $d_1 = 0{,}01$ ml/min.

Après plus de 150 heures de fonctionnement continu l'efficacité de la colonne de catalyse maintenue à 29-30° C demeure inaltérée.

Le rendement déterminé par dosage de l'acide aminé soutiré est de 96%.

Pendant cette période on recueille:
150×60×0,1 = 900 ml de solution d'alaninate de sodium 0,863M.

*EXEMPLE II:*

*PRÉPARATION DE LA MÉTHIONINE EN CONTINU, À PARTIR DE L'α-HYDROXYMÉTHYLMERCAPTO-BUTYRONITRILE:*

Le procédé est le même que dans le cas de l'alanine (exemple I). Toutefois, l'α-amino-méthylmercapto butyronitrile molaire est obtenu par

chauffage à 45° C pendant 1 heure 30 min. de l'α-hydroxyméthylmercapto butyronitrile dans l'ammoniaque 15N. On le stocke à température de 35° C, et l'injecte à débit de 0,06 ml/minute dans la colonne de catalyse thermostatée à 37° C et contenant 2 g de résine pipéridone ($C_{pC=O}$ = 2,4 meq./g).

L'α-amino-nitrile est dilué à l'entrée de la colonne de catalyse par la solution d'α-amino-acide basique 1M, et 0,15N en soude recyclée avec un débit de 0,92 ml/minute. Le réacteur d'hydrolyse est maintenu à 90° C.

Le rendement déterminé par dosage du groupement carboxylique de l'acide aminé est de 95%.

Le contrôle du fonctionnement est effectué par RMN. Au pH auquel on opère, le signal du groupement méthyle de l'α-amino-nitrile, et de l'α-amino-amide sont suffisamment séparés pour permettre de contrôler la réaction d'hydratation.

*EXEMPLE III:*

*COMPARAISON «PROCÉDÉ CATALYTIQUE» – PROCÉDÉ BUCHERER:*

Pour mieux situer l'efficacité du procédé catalytique, il a été jugé intéressant de le comparer au procédé «BUCHERER» actuellement utilisé industriellement pour la synthèse de la méthionine, et qui est basé sur la réaction de Bucherer-Bergs.

Ces deux procédés, dont les phases essentielles sont regroupées dans le tableau 1, passent tous deux par le même intermédiaire réactionnel, à savoir l'α-amino-nitrile, et conduisent au sel de sodium de l'α-amino-acide soit par l'intermédiaire de l'hydantoïne (procédé Bucherer), soit par l'intermédiaire de l'α-amino-amide (procédé catalytique).

Dans le procédé catalytique l'α-amino-nitrile est préalablement synthétisé avant d'être hydraté catalytiquement. Par contre, dans le procédé Bucherer les deux étapes de formation de l'α-amino-nitrile, et de l'hydantoïne sont effectuées dans le même milieu réactionnel, ce qui ne présente pas que des avantages. En effet, pour obtenir une vitesse de formation de l'hydantoïne satisfaisante, il faut se placer dans des conditions de température relativement élevées, qui favorisent thermodynamiquement les produits de décomposition de l'α-amino-nitrile.

La comparaison portera sur quatre aspects importants de ces deux procédés:
- rendement,
- réactivité,
- bilan global matière,
- recyclage des intermédiaires de synthèse, successivement analysés ci-après.

1/ *Rendement:*

Il a été constaté que dans l'installation pilote décrite ci-dessus le rendement en sel de sodium de l'alanine par rapport à l'acétaldéhyde de départ est de l'ordre de 96%. Dans le procédé Bucherer le rendement également en sel de sodium de l'alanine se situe selon les cas entre 85 et 90% par rapport à l'acétaldéhyde.

*Tableau 1*

*Bilan et Recyclage du «Procédé catalytique»*

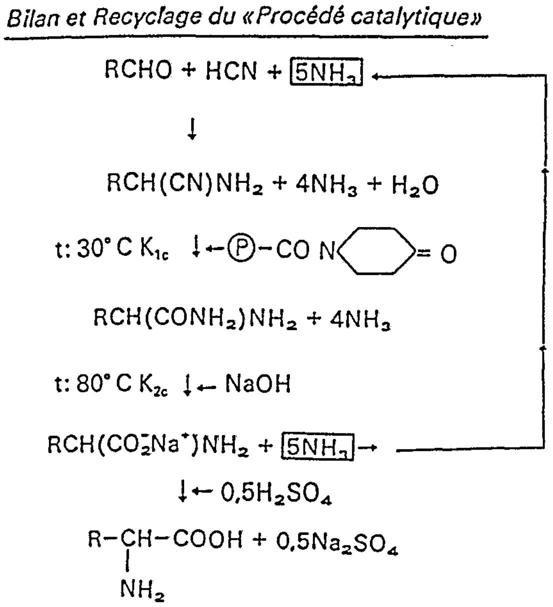


*Bilan théorique en régime stationnaire*

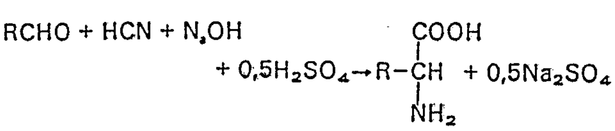

*Bilan et Recyclage du «Procédé Bucherer»*

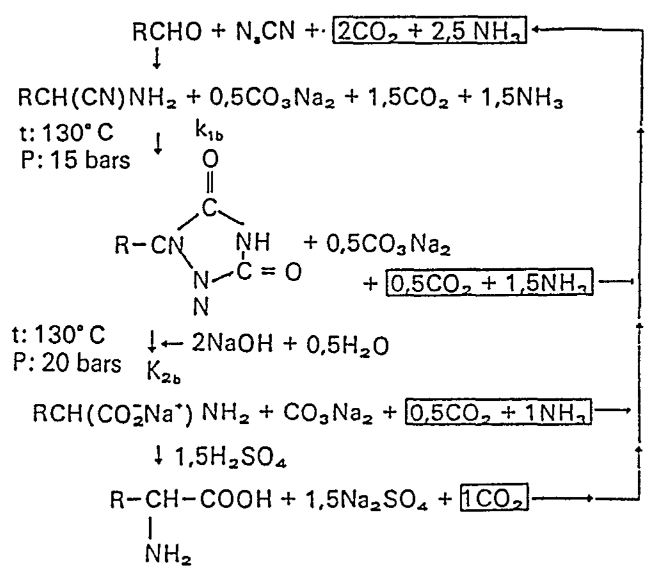


*Bilan théorique en régime stationnaire*

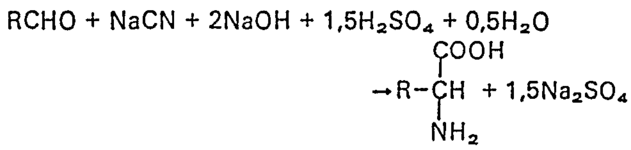

2/ *Réactivité:*

La comparaison cinétique des deux procédés peut être conduite depuis l'α-amino-nitrile intermédiaire commun aux deux processus, jusqu'à la formation du sel de sodium de l'acide aminé.

La vitesse de chacun des processus est réglée d'une part par les vitesses de formation ($k_{1B}$) et d'hydrolyse ($k_{2B}$) de l'hydantoïne, et d'autre part par les vitesses de formation ($k_1c$) et d'hydrolyse ($k_2C$) de l'α-amino-amide.

Les valeurs de ces constantes de vitesse seront comparées uniquement dans le cas de l'alanine, mais on sait qu'elles sont représentatives, à un ordre de grandeur près, de la réactivité des précurseurs de la méthionine.

. *Formation de l'hydantoïne — $k_1B$:*

La vitesse de formation de l'hydantoïne est du premier ordre par rapport à la concentration en carbamate de l'α-amino-nitrile. En faisant l'hypothèse (techniquement irréalisable) que l'on se place dans les conditions (pression partielle de $CO_2$, pH, décomposition de l'α-amino-nitrile négligeable) les plus favorables pour lesquelles l'α-amino-nitrile est entièrement sous forme de carbamate, les valeurs de la constante de vitesse $k_1B$ d'apparition de l'hydantoïne en fonction de la température sont:

| t | $k_1B$ $min^{-1}$ |
|---|---|
| 30° C | $0{,}028.10^{-2}$ |
| 50° C | $0{,}4.10^{-2}$ |
| 75° C | $5.10^{-2}$ |

$E_a = 23$ kcal/mole

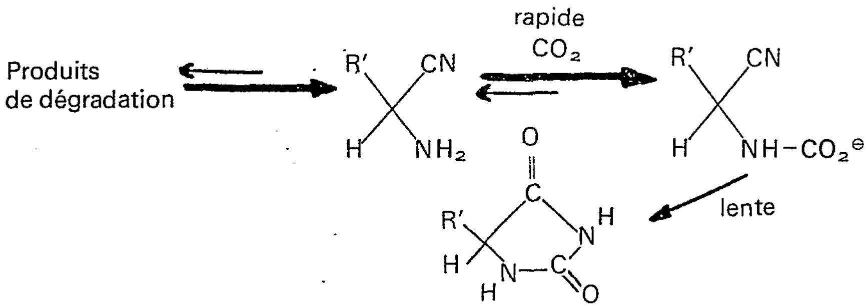


. *Hydrolyse de l'hydantoïne $k_2B$*

La vitesse de cette réaction est du premier ordre par rapport à la concentration en hydantoïne. En milieu suffisamment basique ($[OH^-] = 0{,}1N$), l'hydantoïne *a* est de façon prédominante sous forme ionique *b*. L'étape lente de la réaction d'hydrolyse indépendante du pH du milieu réactionnel, est l'attaque de $OH^-$ sur l'hydantoïne non ionisée:

| | t | $k_2B$ $mn^{-1}$ |
|---|---|---|
| $R' = CH_3$ | 60° C | $6{,}5\ 10^{-3}$ |
| | 80° C | $3\ 10^{-2}$ |

$E_A = 18$ Kcal/mole

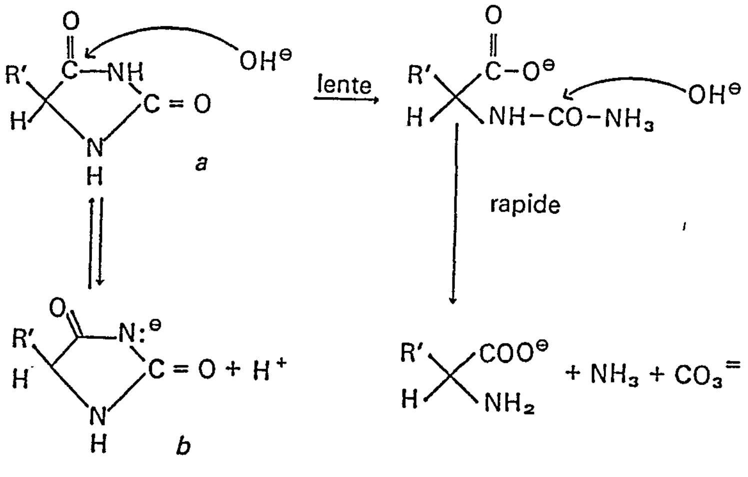


. *Hydratation catalytique de l'α-amino-nitrile $k_1C$:*

La vitesse d'hydratation catalytique de l'α-amino-nitrile, proportionnelle à la masse de résine carbonylée est du premier ordre par rapport à la concentration en α-amino-nitrile et en ions hydroxyles. On a ainsi pu déterminer lors d'essais en «batch» les valeurs de constante de vitesse à différentes températures.

R' = $CH_3$

| t | $K_1C\ M^{-1}\ min^{-1}$ (par gramme de résine) | |
|---|---|---|
| 8° C | 3,26 | |
| 20° C | 4,08 | $E_A$ = 3 Kcal/mole. |
| 34° C | 5,31 | |

. *Hydrolyse basique de l'α– amino-amide - $k_2C$:*

Cette réaction est analogue du point de vue mécanistique à l'hydrolyse basique des amides monofonctionnels. Elle est du premier ordre par rapport aux concentrations en α-amino-amide, et en ions hydroxyles

| t | $k_2C\ M^{-1}\ min^{-1}$ | |
|---|---|---|
| 15° C | 0,0063 | $E_A$ = 13,5 Kcal/mole. |
| 35° C | 0,029 | |
| 80° C | 0,36 | |

Le tableau ci-dessous regroupe les valeurs des différentes constantes de vitesse, précédemment définies.

| Procédé bucherer | Procédé catalytique |
|---|---|
| $k_1B$: 0,05 $min^{-1}$ (75° C) | $k_1C = 4\ M^{-1}min^{-1}$/g de résine (30° C) |
| $k_2B$: 0,03 $min^{-1}$ (80° C) | $k_2C = 0,36\ M^{-1}min^{-1}$ (80° C) |

La comparaison directe des valeurs numériques des constantes de vitesse $k_1B$ et $k_1C$ est relativement délicate. Il est cependant évident que la formation de l'α-amino-amide effectuée à température ambiante (Ea pratiquement nulle) est nettement plus rapide que celle de l'hydantoïne.

Ainsi, le temps de ½ formation de l'hydantoïne à 75° C est de l'ordre de 15 min (valeur théorique optimale ne tenant pas compte de la décomposition équilibrée de l'α-amino-nitrile).

Dans des conditions représentatives ([nitrile] = [$OH^-$] = 0,05M, masse de résine 1,5 g $Cp_{C=O}$= 1,4 méq./g volume de solution 15 $cm^3$ t=20°) le temps de ½ réaction d'hydratation de l'α-amino-nitrile n'est que de 2 minutes.

Les valeurs des constantes de vitesse $k_2C$ et $k_2B$ de formation de l'alaninate de sodium, dans la soude 1N sont, elles, directement comparables. On constate que dans les conditions précisées (80° C) l'hydrolyse de l'α-amino-amide est 10 fois plus rapide que celle de l'hydantoïne. Cet écart s'atténue toutefois à température plus élevée, en raison de la différence observée entre les énergies d'activation des deux réactions.

3/ *Bilan global de matière:*

Le bilan matière est nettement en faveur du procédé catalytique. Par mole d'acide aminé produit n'est formé en effet que 0,5 mole de $SO_4Na_2$ provenant exclusivement de la neutralisation de l'alaninate de sodium. Par contre, le procédé Bucherer, qui utilise comme réactif NaCN au lieu de HCN, conduit, par mole d'acide aminé produit, à la formation de 1,5 mole de $SO_4Na_2$, provenant de la neutralisation de l'alaninate de sodium, et du carbonate de sodium formé lors de l'hydrolyse de l'hydantoïne.

Cette comparaison, quant à la formation de sous-produits non valorisables ($SO_4Na_2$), revêt toute sa signification, si l'on prend en considération l'important tonnage de méthionine actuellement produit par le procédé Bucherer.

4/ *Recyclage des intermédiaires de synthèse:*

Si les avantages du procédé catalytique apparaissent clairement tant au niveau réactivité que quantité de sous-produits formés, la simplicité de fonctionnement par rapport au procédé Bucherer est encore plus évidente.

Le procédé catalytique ne nécessite qu'un seul recyclage relativement facile de l'ammoniac, alors que le procédé Bucherer implique trois recyclages successifs de deux produits ($CO_2$ et $NH_3$) au niveau des trois étapes réactionnelles.

Cette analyse démontre clairement les avantages liés au procédé objet de la présente invention.

## Revendications

1. Procédé de synthèse en continu d'un α-amino-acide par hydrolyse catalytique chimique en milieu basique aqueux, d'un α-amino-nitrile ou d'un de ses sels, en présence d'au moins un dérivé carbonylé, comportant:

a) une première étape d'hydratation catalytique de l'α-amino-nitrile de départ ou l'un de ses sels, en l'α-amino-amide correspondant, en présence d'une faible concentration d'ions hydroxydes et en utilisant en tant que catalyseur une résine polymère carbonylée insoluble en milieu basique aqueux,

b) une deuxième étape d'hydrolyse de l'α-amino-amide ainsi formé en α-amino-acide sous la forme d'un de ses sels en présence d'ions hydroxydes en concentration sensiblement équimolaire par rapport à la concentration de l'α-amino-amide,

caractérisé en ce qu'on prélève une fraction du volume du milieu réactionnel de la deuxième étape en vue de son recyclage, après refroidissement, vers le milieu réactionnel de la première étape, ladite fraction étant déterminée de façon à assurer la dilution de l'α-amino-nitrile ou l'un de ses sels, introduit dans le milieu de la première étape, et à maintenir en dessous du seuil d'empoisonnement de la résine polymère carbonylée, la concentration en α-amino-amide du milieu réactionnel de la première étape, ledit seuil d'empoisonnement étant égal à environ 0,20 M/l d'α-amino-amide.

2. Procédé de synthèse d'un α-amino-acide, sous la forme de l'un de ses sels, à une concentration de xM à partir d'un α-amino-nitrile, ou d'un de ses sels, à la même concentration xM, selon la revendication 1, caractérisé en ce que la fraction du volume du milieu réactionnel de la deuxième étape, prélevée et recyclée, est déterminée de manière à ce que la concentration en α-amino-amide,

dans le milieu de la première étape, soit sensiblement de $\frac{x}{10}$ M.

3. Procédé de synthèse d'un α-amino-acide, sous la forme de l'un de ses sels, à une concentration de 1 M à partir d'un α-amino-nitrile, ou d'un de ses sels, à la même concentration 1 M, selon la revendication 2, caractérisé en ce que la fraction du volume du milieu réactionnel de la deuxième étape, prélevée et recyclée est déterminée de manière à ce que la concentration en α-amino-amide, dans le milieu réactionnel de la première étape, soit sensiblement décimolaire.

4. Procédé de synthèse selon l'une des revendications 1 à 3, caractérisé en ce que la quantité d'ions hydroxydes nécessaires à la catalyse dans la première étape provient du recyclage d'une fraction du volume réactionnel de la deuxième étape.

5. Dispositif pour la mise en œuvre du procédé de synthèse en continu d'un α-amino-acide selon l'une des revendications 1 à 4, comportant:

a) une cuve d'alimentation (10) en α-amino-nitrile, ou en l'un de ses sels,
b) un conduit de raccordement (12) sur lequel est montée une pompe (14), reliant la cuve d'alimentation (10) à
c) une colonne de catalyse (16) contenant une résine polymère carbonylée (18) insoluble en milieu basique aqueux,
d) un conduit de raccordement (20) de la colonne de catalyse (16) à
e) un réacteur d'hydrolyse (22), faisant simultanément office de réservoir pour le sel d'α-amino-acide formé, comportant des moyens de chauffage et d'agitation du milieu réactionnel, et muni
f) de moyens d'alimentation en ions hydroxydes comprenant une cuve d'alimentation (24), un conduit (26) et une pompe (28),
g) d'un conduit de soutirage (30) du sel d'α-amino-acide formé,

caractérisé en ce que ledit dispositif comporte, en outre, un conduit de recyclage (32) équipé d'un réfrigérant (34) et d'une pompe (36), ledit conduit de recyclage raccordant le réacteur d'hydrolyse (22) à la colonne de catalyse (16) afin d'assurer la dilution du courant d'α-amino-nitrile, ou d'un de ses sels, alimentant ladite colonne de catalyse.

6. Dispositif selon la revendication 5, caractérisé en ce que les pompes (14, 28, 36) respectivement montées sur les conduits

– d'alimentation en α-amino-nitrile, ou l'un de ses sels (12),
– d'alimentation en ions hydroxydes (26), et
– de recyclage (32) du milieu réactionnel de la deuxième étape,

sont des pompes centrifuges à débits réglables.

7. Dispositif selon l'une des revendications 5 et 6, caractérisé en ce que le réacteur d'hydrolyse (22) possède en outre un conduit (38) débouchant dans le ciel gazeux dudit réacteur et destiné à recycler l'ammoniac dégagé lors de la deuxième étape en vue de son utilisation pour la préparation in situ de l'α-amino-nitrile de départ, ou l'un de ses sels.

8. Dispositif selon l'une des revendications 5 à 7, caractérisé en ce qu'il comporte au moins une deuxième colonne de catalyse montée en parallèle par rapport à la première colonne (16) pour fonctionner en alternance par rapport à celle-ci, chacune de ces colonnes étant équipée de moyens aptes à régénérer la résine polymère carbonylée (18), et d'un système de vannes permettant le fonctionnement alternatif des colonnes de catalyse.

9. Dispositif selon la revendication 8, caractérisé en ce que lesdits moyens aptes à régénérer la résine polymère carbonylée comportent un circuit de lavage à l'eau comprenant (i) une arrivée d'eau chauffée à environ 80° C, (ii) une colonne de régénération dans laquelle est disposée une résine sulfonique pour fixer l'amide entraîné dans ledit circuit, ladite colonne de régénération étant raccordée par (iii) des conduits de raccordement à l'entrée et à la sortie de la colonne de catalyse.

## Claims

1. A process for the continuous synthesis of an α-amino acid by chemical catalytic hydrolysis in an aqueous basic medium of an α-amino-nitrile or of one of its salts, in the presence of at least one carbonyl derivative, comprising

a) a first stage involving catalytic hydration of the starting α-amino-nitrile or one of its salts, into the corresponding α-amino amide, in the presence of a weak concentration of hydroxide ions and using a carbonyl polymeric resin which is insoluble in an aqueous basic medium as catalyst,
b) a second stage involving hydrolysis of the α-amino amide thus formed into α-amino acid in the form of one of its salts in the presence of hydroxide ions in a concentration which is substantially equimolar relative to the concentration of the α-amino-amide,

characterised in that a fraction of the volume of the reaction medium from the second stage is removed so that it can be re-cycled, after cooling, to the reaction medium from the first stage, said fraction being determined so as to allow dilution of the α-amino-nitrile or one of its salts, introduced into the medium from the first stage, and to maintain the α-amino amide concentration of the reaction medium from the first stage below the poison threshold of the carbonyl polymeric resin, said poison threshold being equal to approximately 0.20 M/l of α-amino amide.

2. A process for the synthesis of an α-amino acid in the form of one of its salts, at a concentration of xM from an α-amino nitrile or one of its salts at the same concentration xM according to claim 1, characterised in that the removed and recycled fraction of reaction medium from the second stage is determined such that the α-amino amide concentration in the medium from the first stage is substantially $\frac{x}{10}$ M.

3. A process for the synthesis of an α-amino acid in the form of one of its salts at a concentration

of 1 M from an α-amino nitrile or one of its salts at the same concentration 1 M according to claim 2, characterised in that the removed and re-cycled fraction of the volume of the reaction medium from the second stage is determined such that the concentration of α-amino amide in the reaction medium from the first stage is substantially decimolar.

4. A synthesis process according to one of claims 1 to 3, characterised in that the quantity of hydroxide ions required for catalysis in the first stage originates from the re-cycling of a fraction of the reaction volume from the second stage.

5. A device for carrying out the process for the continuous synthesis of an α-amino acid according to one of claims 1 to 4, comprising

a) a supply tank (10) for the supply of α-amino nitrile or one of its salts,
b) a connecting conduit (12) on which there is mounted a pump (14) connecting the supply tank (19) to
c) a catalysis column (16) containing a carbonyl polymeric resin (18) which is insoluble in an aqueous basic medium,
d) a connecting conduit (20) for connecting the catalysis column (16) to
e) a hydrolysis reactor (22) simultaneously acting as a reservoir for the α-amino acid salt formed and comprising means for heating and stirring the reaction medium and provided with
f) hydroxide ion supply means comprising a supply tank (24), a conduit (26) and a pump (28),
g) an extraction conduit (30) for extracting the α-amino acid salt formed,

characterised in that said device also comprises a re-cycling conduit (32) equipped with a refrigerant (34) and a pump (36), said re-cycling conduit connecting the hydrolysis reactor (22) to the catalysis column (16) to allow dilution of the stream of α-amino nitrile or of one of its salts supplying said catalysis column.

6. A device according to claim 5, characterised in that the pumps (14, 28, 36) respectively mounted on the conduits

– for supply of α-amino nitrile or of one of its salts (12),
– for supply of hydroxide ions (26), and
– for re-cycling (32) of the reaction medium from the second stage,

are adjustable output centrifugal pumps.

7. A device according to one of claims 5 and 6, characterised in that the hydrolysis reactor (22) also has a conduit (38) opening into the gaseous top of said reactor and intended to re-cycle the ammonia released during the second stage so that it can be used for the preparation in situ of the starting α-amino nitrile or one of its salts.

8. A device according to one of claims 5 to 7, characterised in that it comprises at least one second catalysis column mounted in parallel with the first column (16) to operate in alternation therewith, each of these columns being equipped with means for regenerating the carbonyl polymeric resin (18) and with a valve system allowing alternate operation of the catalysis columns.

9. A device according to claim 8, characterised in that said means for regenerating the carbonyl polymeric resin comprise a water washing circuit consisting of (i) an inlet for water heated to about 80° C, (ii) a regeneration column containing a sulphonic resin for fixing the amide entrained in said circuit, said regenerating column being connected via (iii) connecting conduits to the inlet and outlet of the catalysis column.

## Patentansprüche

1. Verfahren zum kontinuierlichen Herstellen einer α-Aminosäure durch katalytische chemische Hydrolyse eines α-Aminonitrils oder eines seiner Salze in wässerigem basischem Medium in Gegenwart mindestens einer Verbindung mit Carbonylgruppen mit

a) einer ersten Stufe der katalytischen Hydratation des α-Aminonitrils oder eines seiner Salze als Ausgangsmaterial zu dem entsprechenden α-Aminoamid in Gegenwart einer geringen Konzentration von Hydroxylionen und unter Verwendung eines in dem wässerigen basischen Medium unlöslichen, Carbonylgruppen enthaltenden polymeren Harzes als Katalysator und
b) einer zweiten Stufe der Hydrolyse des gebildeten α-Aminoamids zur α-Aminosäure in Form eines seiner Salze in Gegenwart von Hydroxylionen in einer zur Konzentration des α-Aminoamids etwa equimolaren Konzentration,

dadurch gekennzeichnet, dass man aus dem Reaktionsmedium der zweiten Stufe einen Anteil seines Volumens abzieht, um ihn nach dem Kühlen in das Reaktionsmedium der ersten Stufe zurückzuführen, wobei der genannte Anteil dahingehend bestimmt wird, die Verdünnung des α-Aminonitrils oder eines seiner Salze, das in das Medium der ersten Stufe eingeführt wird, sicherzustellen und die Konzentration des α-Aminoamids im Reaktionsmedium der ersten Stufe unter der Vergiftungsschwelle des Carbonylgruppen enthaltenden polymeren Harzes zu halten, wobei die genannte Vergiftungsschwelle etwa 0,20 Mol/l an α-Aminoamid beträgt.

2. Verfahren zum Herstellen einer α-Aminosäure in Form eines ihrer Salze in einer Konzentration von x Mol, ausgehend von einem α-Aminonitril oder einem seiner Salze in der gleichen Konzentration x Mol nach Anspruch 1, dadurch gekennzeichnet, dass der Volumenanteil des Reaktionsmediums der zweiten Stufe, der entnommen und zurückgeführt wird, so bestimmt wird, dass die Konzentration an α-Aminoamid im Medium der ersten Stufe etwa $\frac{x}{10}$ Mol beträgt.

3. Verfahren zum Herstellen einer α-Aminosäure in Form eines ihrer Salze in einer Konzentration von 1 Mol, ausgehend von einem α-Aminonitril oder einem seiner Salze in der gleichen Konzentration von 1 Mol nach Anspruch 2, dadurch gekennzeichnet, dass der Volumenanteil des Reaktionsmediums der zweiten Stufe, der entnommen und

zurückgeführt wird, so bestimmt wird, dass die Konzentration an α-Aminoamid in Reaktionsmedium der ersten Stufe etwa dezimolar ist.

4. Verfahren nach einem der Ansprüche 1 bis 3, dadurch gekennzeichnet, dass die Menge an Hydroxylionen, die zur Katalyse in der ersten Stufe erforderlich ist, aus der Rückführung eines Volumenanteils des Reaktionsmediums der zweiten Stufe stammt.

5. Vorrichtung zur Durchführung des Verfahrens zum kontinuierlichen Herstellen einer α-Aminosäure nach einem der Ansprüche 1 bis 4 mit:

a) einem Behälter (10) zur Zufuhr von α-Aminonitril oder eines seiner Salze,
b) einer Leitung (12) mit einer Pumpe (14), die den Zufuhrbehälter (10) mit
c) einer Katalysesäule (16) verbindet, die ein Carbonylgruppen aufweisendes polymeres Harz (18) enthält, das in wässerigem, basischem Medium unlöslich ist,
d) einer Verbindungsleitung (20) von der Katalysesäure (16) zu
e) einem Hydrolysereaktor (22), der gleichzeitig als Reservoir für das gebildete Salz der α-Aminosäure dient, Einrichtungen zum Erhitzen und Rühren des Reaktionsmedium umfasst und versehen ist mit
f) einer Zufuhreinrichtung für Hydroxylionen, umfassend einen Zufuhrbehälter (24), eine Leitung (26) und eine Pumpe (28) sowie
g) einer Leitung (30) zum Abziehen des gebildeten Salzes der α-Aminosäure,

dadurch gekennzeichnet, dass die Vorrichtung ausserdem eine mit einem Kühlaggregat (34) und einer Pumpe (36) ausgerüstete Rückführleitung (32) umfasst, die den Hydrolysereaktor (22) mit der Katalysesäule (16) verbindet, um den Strom des α-Aminonitrils oder eines seiner Salze, der der Katalysesäule zugeführt wird, zu verdünnen.

6. Vorrichtung nach Anspruch 5, dadurch gekennzeichnet, dass die Pumpen (14, 28, 36), die jeweils in einer der folgenden Leitungen montiert sind

– für die Zufuhr von α-Aminonitril oder eines seiner Salze (12),
– die Zufuhr von Hydroxylionen (26) und
– die Rückführung (32) des Reaktionsmedium der zweiten Stufe,

Zentrifugalpumpen mit regelbarer Förderleistung sind.

7. Vorrichtung nach einem der Ansprüche 5 und 6, dadurch gekennzeichnet, dass der Hydrolysereaktor (22) ausserdem eine vom gasgefüllten Oberteil des genannten Reaktors ausgehende Leitung (38) aufweist, die bestimmt ist, das in der zweiten Stufe freigesetzte Ammoniak zurückzuführen zum Einsatz für die in situ-Herstellung des als Ausgangsmaterial dienenden α-Aminonitrils oder eines seiner Salze.

8. Vorrichtung nach einem der Ansprüche 5 bis 7, dadurch gekennzeichnet, dass sie mindestens eine zweite Katalysesäule umfasst, die parallel zur ersten Säule (16) montiert ist, um sie alternierend damit einzusetzen, wobei jede der Säulen mit Einrichtungen ausgerüstet ist, um das Carbonylgruppen aufweisende polymere Harz (18) zu regenerieren und ein Ventilsystem vorhanden ist, das die alternative Benutzung der Katalysesäulen gestattet.

9. Vorrichtung nach Anspruch 8, dadurch gekennzeichnet, dass die Einrichtung zum Regenerieren des Carbonylgruppen aufweisenden polymeren Harzes einen Kreislauf zum Waschen mit Wasser umfasst mit

(i) einer Zufuhr von auf etwa 80° C erhitztem Wasser,
(ii) einer Regenerationssäule, in der ein sulfoniertes Harz angeordnet ist, um in dem Kreislauf mitgeführtes Amid festzulegen und
(iii) Verbindungsleitungen die die Regenerationssäule mit dem Eingang und dem Ausgang der Katalysesäule verbinden.

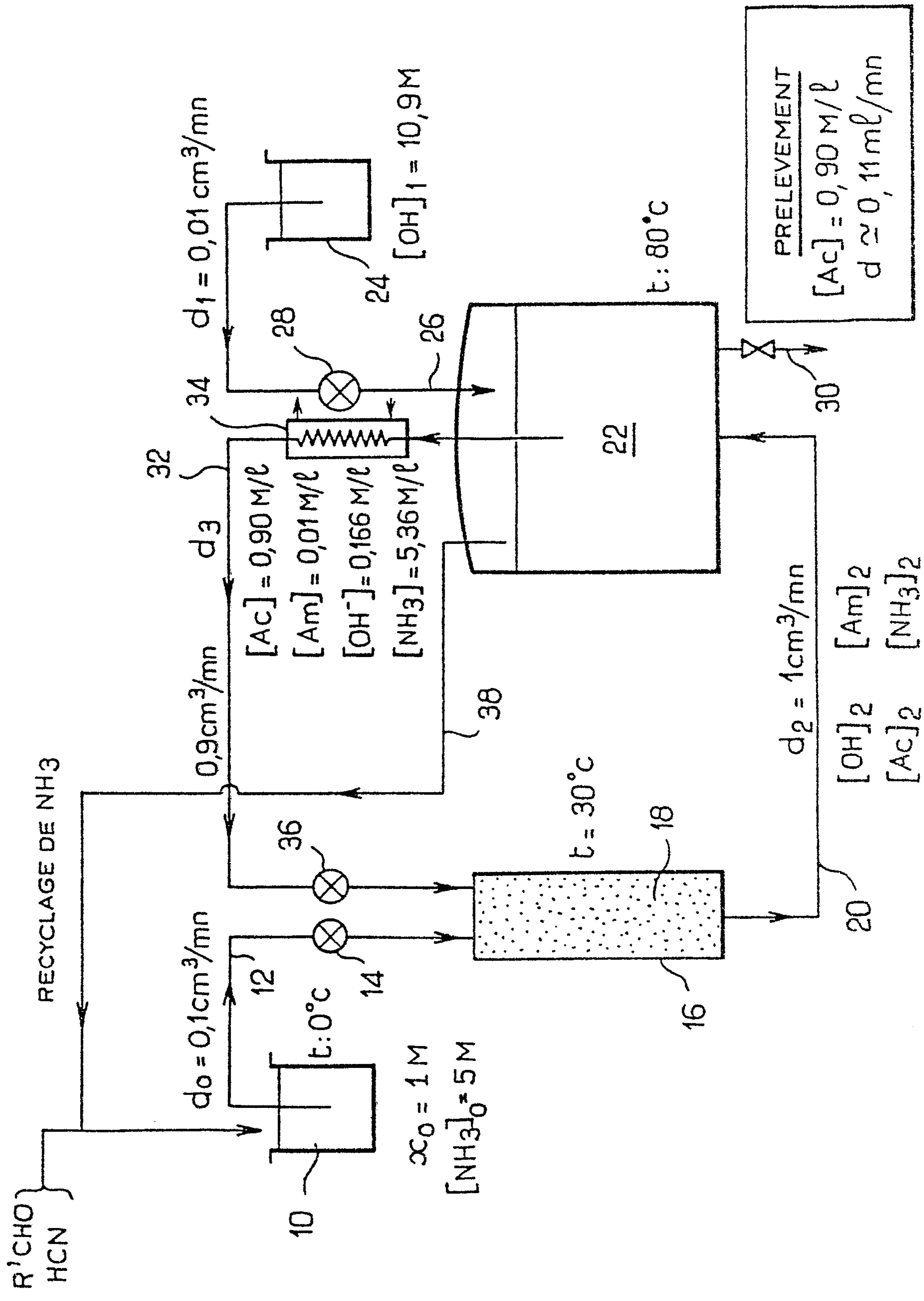
R'CHO
HCN
RECYCLAGE DE NH3
10
$x_o$ = 1 M
$[NH_3]_o$ = 5 M
t: 0°c
$d_o$ = 0,1cm³/mn
12
14
36
0,9cm³/mn
$d_3$
32
34
28
26
$d_1$ = 0,01 cm³/mn
24
$[OH]_1$ = 10,9M
[Ac] = 0,90 M/ℓ
[Am] = 0,01 M/ℓ
$[OH^-]$ = 0,166 M/ℓ
$[NH_3]$ = 5,36 M/ℓ
38
t = 30°c
18
16
20
22
t: 80°c
30
$d_2$ = 1cm³/mn
$[OH]_2$ $[Am]_2$
$[Ac]_2$ $[NH_3]_2$
PRELEVEMENT
[Ac] = 0,90 M/ℓ
d ≃ 0,11mℓ/mn